# FASCICULE DE BREVET EUROPEEN

(11) **EP 2 301 630 B1**
(45) Date de publication et mention de la délivrance du brevet: **24.10.2018**
(21) Numéro de dépôt: 09179777.9
(22) Date de dépôt: 18.12.2009
(51) Int. Cl.: A61Q 5/04, A61Q 5/08, A61Q 5/10, A61K 8/22, A61K 8/42, A61K 8/81, A61K 8/84, A61K 8/45, A61K 8/46, A61K 8/67

(54) **Composition oxydante pour le traitement des fibres kératiniques comprenant un polymère cationique, un amide gras et un agent anti-oxygène**
Haarzusammensetzung enthaltend kationisches Polymer, ein Fettamid und ein Antioxidant
Oxidizing composition for the treatment of keratin fibres comprising a cationic polymer, a fatty amide and an anti-oxygen agent

(30) Priorité: 19.12.2008 FR 0858838
(43) Date de publication de la demande: 30.03.2011
(73) Titulaire: L'Oréal, 75008 Paris (FR)
(72) Inventeur: Braida-Valerio, Damarys, 75012, Paris (FR); Nodari, Laurent, 95100, Argenteuil (FR); Cotteret, Jean, 78600, Maisons Laffite (FR)
(74) Mandataire: Prevel, Estelle Nicole

(56) Documents cités:
- WO-A-03/053329
- CA-A1- 1 268 421
- DE-A1- 4 309 509
- GB-A- 2 170 830
- US-A- 4 226 851

## Description

La présente invention a pour objet une composition pour le traitement des fibres kératiniques, et en particulier des fibres kératiniques humaines telles que les cheveux, comprenant, dans un milieu cosmétiquement acceptable, un ou plusieurs polymères cationiques ; un ou plusieurs amides gras; un ou plusieurs agents anti-oxygène ; un ou plusieurs agents oxydants et au moins 10 % en poids d'une ou plusieurs huiles.

En cosmétique, dans les domaines de la teinture, de la décoloration et de la déformation permanente des fibres kératiniques, et en particulier des fibres kératiniques humaines telles que les cheveux, on utilise des compositions oxydantes.

Ainsi, en teinture d'oxydation des cheveux, des compositions oxydantes sont mélangées aux colorants d'oxydation (bases et coupleurs), qui sont incolores par eux-mêmes, pour engendrer des composés colorés et colorants par un processus de condensation oxydative. Des compositions oxydantes sont également utilisées en teinture directe des cheveux en mélange avec certains colorants directs qui sont colorés et colorants pour obtenir une coloration avec un effet éclaircissant des cheveux. Parmi les agents oxydants classiquement utilisés pour la teinture des fibres kératiniques, on peut citer le peroxyde d'hydrogène ou des composés susceptibles de produire le peroxyde d'hydrogène par hydrolyse tels que le peroxyde d'urée. On peut aussi utiliser les persels comme les perborates et persulfates. Le peroxyde d'hydrogène est plus particulièrement préféré.

En décoloration des cheveux, les compositions de décoloration contiennent un ou plusieurs agents oxydants. Parmi ces agents oxydants, les plus classiquement utilisés sont le peroxyde d'hydrogène ou des composés susceptibles de produire le peroxyde d'hydrogène par hydrolyse, tels que le peroxyde d'urée ou les persels comme les perborates, les percarbonates et les persulfates, le peroxyde d'hydrogène et les persulfates étant particulièrement préférés.

Ces compositions peuvent être des compositions aqueuses contenant des agents alcalins (amines ou ammoniaque) que l'on dilue au moment de l'emploi avec une composition aqueuse de peroxyde d'hydrogène.

Ces compositions peuvent aussi être formées de produits anhydres qui contiennent des composés alcalins (amines et / ou silicates alcalins), et un réactif peroxygéné tels que les persulfates, perborates ou percarbonates, d'ammonium ou de métaux alcalins, que l'on dilue au moment de l'emploi avec une composition aqueuse de peroxyde d'hydrogène.

En déformation permanente des cheveux, dans un premier temps, on réalise l'ouverture des liaisons disulfures -S-S- de la kératine (cystine) à l'aide d'une composition contenant un agent réducteur adapté (étape de réduction) puis, après avoir rincé la chevelure ainsi traitée, on reconstitue dans un second temps les liaisons disulfures en appliquant, sur les cheveux préalablement mis sous tension (bigoudis et autres), une composition oxydante (étape d'oxydation, dite aussi de fixation) de façon à donner finalement aux cheveux la forme recherchée. Cette technique permet ainsi de réaliser indifféremment soit l'ondulation des cheveux, soit leur défrisage ou leur décrêpagé. La nouvelle forme imposée aux cheveux par un traitement chimique tel que ci-dessus est éminemment durable dans le temps et résiste notamment à l'action des lavages à l'eau ou des shampooings, et ceci par opposition aux simples techniques classiques de déformation temporaire, telles que de mise en pli.

Les compositions oxydantes nécessaires à la mise en oeuvre de l'étape de fixation, sont le plus souvent des compositions à base d'eau oxygénée.

Par ailleurs, dans le domaine cosmétique, on cherche toujours à améliorer le conditionnement des cheveux, c'est-à-dire notamment à améliorer les propriétés de lissage et de douceur au toucher.

Pour ce faire, il est connu d'utiliser des molécules cationiques, par exemple des polymères cationiques ou des tensioactifs cationiques pour améliorer le conditionnement de la fibre.

Cependant, l'introduction de polymères cationiques dans les compositions oxydantes à base d'eau oxygénée conduit à une déstabilisation de ces compositions.

Le but de la présente invention est de fournir de nouvelles compositions oxydantes qui permettent d'améliorer les propriétés cosmétiques des cheveux et qui sont stables dans le temps.

Ce but est atteint avec la présente invention qui a pour objet une composition pour le traitement des fibres kératiniques, et en particulier des fibres kératiniques humaines telles que les cheveux, comprenant, dans un milieu cosmétiquement acceptable :
- un ou plusieurs polymères cationiques ;
- un ou plusieurs amides gras;
- un ou plusieurs agents anti-oxygène choisis parmi la vitamine E , les éthers ou esters de vitamine E ; et
   - un ou plusieurs agents oxydants,
   la composition comprenant de plus au moins 10 % en poids du poids total de la composition d'une ou plusieurs huiles.

Lorsque la composition conforme à l'invention est utilisée pour la coloration des fibres kératiniques, on obtient de bonnes propriétés tinctoriales, notamment des colorations puissantes, chromatiques, peu sélectives et résistant bien aux diverses agressions que peuvent subir les cheveux telles que les shampooings, la lumière, la sueur et les déformations permanentes, sans altérer les propriétés cosmétiques des fibres kératiniques.

Lorsque la composition conforme à la présente invention est utilisée pour la décoloration des fibres kératiniques, elle permet d'obtenir un bon effet d'éclaircissement de ces fibres sans les dégrader et sans altérer leurs propriétés cosmétiques.

Lorsque la composition conforme à la présente invention est utilisée pour la déformation permanente des fibres kératiniques, elle permet d'obtenir une déformation permanente satisfaisante de ces fibres sans les dégrader et sans altérer leurs propriétés cosmétiques.

De plus, la composition conforme à l'invention présente une bonne stabilité dans le temps, notamment au stockage à des températures élevées, par exemple de l'ordre de 45 °C.

L'invention a aussi pour objet un procédé de traitement des fibres kératiniques, notament un procédé de coloration, de décoloration ou de déformation permanente des fibres kératiniques, mettant en oeuvre cette composition oxydante.

Un autre objet de l'invention est l'utilisation de cette composition oxydante pour le traitement des fibres kératiniques, notamment la coloration, la décoloration ou la déformation permanente des fibres kératiniques.

Dans ce qui suit, sauf indication contraire, les bornes des intervalles indiqués sont comprises dans l'invention.

Par « polymère cationique », on entend tout polymère contenant des groupements cationiques et / ou des groupements ionisables en groupements cationiques.

Parmi les polymères cationiques, on peut citer plus particulièrement les polymères du type polyamine, polyaminoamide et polyammonium quaternaire.

Les polymères du type polyamine, polyaminoamide, polyammonium quaternaire, que l'on peut utiliser dans la composition de la présente invention, sont notamment décrits dans les brevets français FR 2 505 348 et FR 2 542 997. Parmi ces polymères, on peut citer :
(1) les homopolymères ou copolymères dérivés d'esters ou d'amides de l'acide acrylique ou méthacrylique;
(2) les dérivés de cellulose cationiques tels que :
   (a) les dérivés d'éthers de cellulose comportant des groupements ammonium quaternaire décrits dans le brevet français FR 1 492 597 ;
   (b) les copolymères de cellulose ou les dérivés de cellulose greffés avec un monomère hydrosoluble d'ammonium quaternaire, et décrits notamment dans le brevet US 4 131 576, tels que les hydroxyalkylcelluloses, comme les hydroxyméthyl-, hydroxyéthyl- ou hydroxypropyl-celluloses greffées notamment avec un sel de méthacryloyléthyl-triméthylammonium, de méthacrylamidopropyl-triméthylammonium ou de diméthyldiallylammonium; on peut citer par exemple le polyquaternium 10 (nom INCI);
(3) les autres polysaccharides cationiques décrits plus particulièrement dans les brevets US 3 589 578 et US 4 031 307 tels que les gommes de guar contenant des groupements cationiques trialkylammonium;
(4) les polymères constitués de motifs pipérazinyle et de groupes divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français FR 2 162 025 et FR 2 280 361 ;
(5) les polyaminoamides solubles dans l'eau, tels que ceux notamment décrits dans les brevets français FR 2 252 840 et FR 2 368 508 ;
(6) les dérivés de polyaminoamides, par exemple, les polymères acide adipique / dialkylaminohydroxyalkyldialkylène-triamine dans lesquels le groupe alkyle comporte de 1 à 4 atomes de carbone et désigne de préférence un groupe méthyle, éthyle, propyle, et le groupe alkylène comporte de 1 à 4 atomes de carbone, et désigne de préférence le groupe éthylène. De tels polymères sont notamment décrits dans le brevet français FR 1 583 363 ;
(7) les polymères obtenus par réaction d'une polyalkylène-polyamine comportant deux groupements amine primaire et au moins un groupement amine secondaire, avec un acide dicarboxylique choisi parmi l'acide diglycolique et les acides dicarboxyliques aliphatiques saturés ayant de 3 à 8 atomes de carbone. Le rapport molaire entre la polyalkylène-polylamine et l'acide dicarboxylique étant compris entre 0,8 : 1 et 1,4 : 1 ; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains US 3 227 615 et US 2 961 347 ;
(8) les cyclopolymères d'alkyldiallylamine ou de dialkyldiallylammonium tels que l'homopolymère de chlorure de diméthyldiallyl-ammonium et les copolymères de chlorure de diallyldiméthylammonium et d'acrylamide;
(9) les polymères de diammonium quaternaire présentant une masse moléculaire moyenne en nombre généralement comprise entre 1000 et 100000, tels que, ceux décrits, par exemple, dans les brevets français FR 2 320 330, 2 270 846, 2 316 271, 2 336 434 et 2 413 907 et les brevets US 2 273 780, 2 375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4 026 945 et 4 027 020; on peut citer par exemple le chlorure d'hexadiméthrine (nom INCI), commercialisé par la société CHIMEX sous la référence MEXOMERE PO ;
(10) les polymères de polyammonium quaternaire tels que ceux notamment décrits dans la demande de brevet EP-A-122 324 ;
(11) les polymères quaternaires de vinylpyrrolidone et de vinylimidazole tels que, par exemple, les produits commercialisés sous les dénominations Luviquat® FC 905, FC 550 et FC 370 par la société B.A.S.F;
(12) les polyamines comme le Polyquart® H vendu par HENKEL, référencées sous le nom de " POLYETHYLENEGLYCOL (15) TALLOW POLYAMINE " dans le dictionnaire CTFA ;
(13) les polymères réticulés de sels de méthacryloyloxyalkyl(C₁-C₄) trialkyl(C₁-C₄)ammonium tels que ceux commercialisés sous le nom de SALCARE® SC 92, SALCARE® SC 95 et SALCARE® SC 96 par la Société ALLIED COLLOIDS ; et leurs mélanges.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

De préférence, les polymères cationiques sont choisis parmi les polymères tels que définis aux points (8) et (9), et en particulier le chlorure d'hexadiméthrine et les homo ou copolymères de chlorure de diméthyldiallylammonium. Encore plus préférentiellement, le ou les polymères cationiques sont choisis parmi le chlorure d'hexadiméthrine et le chlorure de poly diméthyldiallylammonium.

Selon un mode de réalisation particulier de l'invention, la composition oxydante comprend deux polymères cationiques.

Selon un mode de réalisation particulier de l'invention, le ou les polymères cationiques ont une densité de charge cationique supérieure à 3 meq./g, de préférence comprise entre 3,5 et 8,5 meq./g. La densité de charge peut être déterminée expérimentalement par la méthode Kjeldahl ou par calcul à partir de la structure du polymère.

Le ou les polymères cationiques représentent généralement de 0,01 à 20 %, de préférence de 0,1 à 10 % en poids par rapport au poids total de la composition selon l'invention.

Par « amide gras », on entend au sens de la présente invention un amide comprenant dans sa structure une chaîne hydrocarbonée comportant au moins 6 atomes de carbone.

Selon un mode de réalisation particulier de l'invention, le ou les amides gras sont choisis parmi les composés de formule (I) suivante : 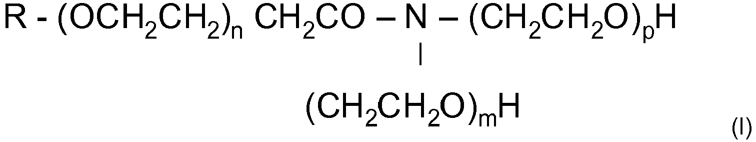 dans laquelle :
- R est un radical alkyle en C₈-C₃₀, saturé ou insaturé, linéaire ou ramifié, éventuellement substitué par un ou plusieurs radicaux choisis dans le groupe constitué par un radical OR₅, un radical NR₆R₇, un radical carboxy, un radical sulfonique, un radical carboxamido CONR₆R₇, un radical sulfonamido SO₂NR₆R₇, un hétéroaryle, un aryle éventuellement substitué par un ou plusieurs groupes (C₁-C₄)alkyle, hydroxy, alcoxy en C₁-C₂, amino, (di)alkyl(C₁-C₂)amino;
- R₅, R₆ et R₇, identiques ou différents, représentent :
   - un atome d'hydrogène ;
   - un radical alkyle linéaire ou ramifié en C₁-C₄ éventuellement substitué par un ou plusieurs radicaux choisis parmi un radical hydroxy, alcoxy en C₁-C₂, carboxamido CONR₈R₉, sulfonyle SO₂R₈, aryle éventuellement substitué par un radical (C₁-C₄)alkyle, hydroxy, alcoxy en C₁-C₂, amino, (di)aikyi(C₁-C₂)amino; aryle éventuellement substitué par un radical (C₁-C₄)alkyle, hydroxy, alcoxy en C₁-C₂, amino, (di)alkyl(C₁-C₂)amino;
- R₆ et R₇, identiques ou différents, peuvent représenter également un radical carboxamido CONR₈R₉; un radical sulfonyle SO₂R₈;
- R₈ et R₉, identiques ou différents, représentent un atome d'hydrogène ; un radical alkyle en C₁-C₄ linéaire ou ramifié éventuellement substitué par un ou plusieurs radicaux hydroxy, alcoxy en C₁-C₂;
- n, m et p, identiques ou différents, sont des nombres entiers allant de 0 à 10, avec m + p > ou= 1.

De préférence, R est un groupe alkyle en C₁₀-C₁₈.

De préférence, n est un nombre entier allant de 0 à 5.

De préférence, m est un nombre entier allant de 0 à 1.

De préférence, p est un nombre entier allant de 1 à 5.

Parmi les composés de formule (I), on peut citer les amides d'acides de colza oxyéthylénés tels que le produit référencé sous le nom INCI PEG-4 RAPESEED AMIDE.

Dans une variante préférée de l'invention, n = 0.

Le ou les amides gras représentent généralement de 0,1 à 20 %, de préférence de 0,2 à 10 %, mieux de 0,5 à 5 % en poids par rapport au poids total de la composition selon l'invention.

Par « agent anti-oxygène », on entend au sens de la présente invention les composés susceptibles d'interagir chimiquement avec l'oxygène.

Cette interaction chimique est cinétiquement très rapide.

Le ou les agents anti-oxygène présents dans la composition conforme à l'invention sont choisis parmi la vitamine E , les éthers ou esters de vitamine E.

Le ou les agents oxydants peuvent être choisis parmi le peroxyde d'hydrogène, les persels comme les persulfates, les percarbonates et perborates, le peroxyde d'urée, les bromates alcalins, les polythionates. Le peroxyde d'hydrogène est préféré.

Le ou les agents oxydants représentent généralement de 0,1 à 50 %, de préférence de 1 à 20 % en poids par rapport au poids total de la composition selon l'invention.

Selon un mode de réalisation particulier de l'invention, lorsque l'agent oxydant est le peroxyde d'hydrogène, la composition oxydante comprend un ou plusieurs agents stabilisants de l'eau oxygénée.

A titre d'exemples d'agents stabilisants de l'eau oxygénée, on peut citer en particulier les pyrophosphates des métaux alcalins ou alcalino-terreux tels que le pyrophosphate de tétrasodium, les stannates des métaux alcalins ou alcalino-terreux, la phénacétine ou les sels d'acides et d'oxyquinoléine comme le sulfate d'oxyquinoféine. De préférence, on utilise un ou plusieurs stannates en association ou non avec un ou plusieurs pyrophosphates.

Le ou les agents stabilisants de l'eau oxygénée représentent généralement de 0,0001 à 5 % en poids, et de préférence de 0,01 à 2 % en poids par rapport au poids total de la composition oxydante.

La composition comprend de plus une ou plusieurs huiles en une teneur d'au moins 10 % en poids du poids total de la composition, pouvant être notamment une huile non siliconée minérale, végétale, animale ou synthétique, ou une huile de silicone. De préférence, la composition comprend une ou plusieurs huiles choisies parmi les huiles non siliconées minérales, végétales, animales ou synthétiques.

Par huile, on entend au sens de la présente invention un composé liquide à 25 °C et à pression atmosphérique (760 mm de mercure) insoluble dans ces conditions dans l'eau à la concentration de 5 %, et de préférence à la concentration de 1%, et comportant au moins deux groupements siloxane ou comportant au moins une chaîne hydrocarbonée comprenant au moins 6 atomes de carbone.

Comme huiles non siliconées utilisables dans la composition de l'invention, on peut citer par exemple :
- les huiles hydrocarbonées d'origine animale, telles que le perhydrosqualèrie;
- les huiles hydrocarbonées d'origine végétale, telles que les triglycérides liquides d'acides gras comportant de 6 à 30 atomes de carbone comme les triglycérides des acides heptanoïque ou octanoïque ou encore, par exemple les huiles noisette, d'abricot, de macadamia, d'arara, de tournesol, de ricin, d'avocat, les triglycérides des acides caprylique / caprique comme ceux vendus par la société Stearineries Dubois ou ceux vendus sous les dénominations Miglyol® 810, 812 et 818 par la société Dynamit Nobel, l'huile de jojoba, l'huile de beurre de karité ;

- les hydrocarbures linéaires ou ramifiés contenant au moins 6 atomes de carbone, d'origine minérale ou synthétique, tels que les alcanes inférieurs C₆-C₁₆ comme l'hexane, le dodécane, l'isododécane et l'isohexadécane, les hydrocarbures à plus de 16 atomes de carbone comme les huiles de paraffine, et leurs dérivés, la vaseline, l'huile de vaseline, les polydécènes, le polyisobutène hydrogéné tel que Parléam®;
- les huiles fluorées partiellement hydrocarbonées ; comme huiles fluorées, on peut citer aussi le perfluorométhylcyclopentane et le perfluoro-1,3 diméthylcyclohexane, vendus sous les dénominations de "FLUTEC® PC1" et "FLUTEC® PC3" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050®" et "PF 5060®" par la Société 3M, ou encore le bromoperfluorooctyle vendu sous la dénomination "FORALKYL®" par la Société Atochem ; le nonafluoro-méthoxybutane et le nonafluoroéthoxyisobutane ; les dérivés de perfluoromorpholine, tels que la 4-trifluorométhyl perfluoromorpholine vendue sous la dénomination "PF 5052®" par la Société 3M.
- les alcools gras liquides en C₇-C₃₀, et en particulier les alcools gras insaturés et / ou ramifiés en C₁₀-C₂₂ tels que l'alcool oléique ou l'alcool isostéarylique, les esters liquides d'acides et / ou d'alcool gras différents des triglycérides, et en particulier ceux pour lesquels l'acide et / ou l'alcool sont insaturés ou ramifiés, on peut utiliser en particulier le myristate d'isopropyle.

Les huiles de silicones peuvent être choisies parmi les polydialkylsiloxanes cycliques comportant de 3 à 7, de préférence de 4 à 5 atomes de silicium. Il s'agit, par exemple, de l'octaméthylcyclotétrasiloxane commercialisé notamment sous le nom de VOLATILE SILICONE® 7207 par UNION CARBIDE ou SILBIONE® 70045 V2 par RHODIA, le décaméthylcyclopentasiloxane commercialisé sous le nom de VOLATILE SILICONE® 7158 par UNION CARBIDE, et SILBIONE® 70045 V5 par RHODIA, ainsi que leurs mélanges.

On peut également citer les cyclocopolymères du type diméthylsiloxanes / méthylalkylsiloxane, tel que la SILICONE VOLATILE® FZ 3109 commercialisée par la société UNION CARBIDE, de formule :

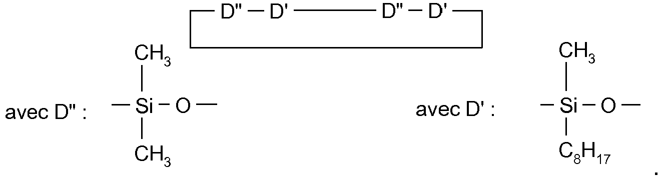

On peut également citer les mélanges de polydialkylsiloxanes cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
- les polydialkylsiloxanes volatiles linéaires ayant 2 à 9 atomes de silicium et présentant une viscosité inférieure ou égale à 5.10⁻⁶ m²/s à 25 °C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and Toiletries, Vol. 91, Jan. 76, P. 27-32 - TODD & BYERS "Volatile Silicone fluids for cosmetics".

On utilise de préférence des polydialkylsiloxanes non volatiles, des gommes et des résines de polydialkylsiloxanes, des polyorganosiloxanes modifiés par les groupements organofonctionnels ci-dessus ainsi que leurs mélanges.

Ces silicones sont plus particulièrement choisies parmi les polydialkylsiloxanes parmi lesquels on peut citer principalement les polydiméthylsiloxanes à groupements terminaux triméthylsilyl. La viscosité des silicones est mesurée à 25 °C selon la norme ASTM 445 Appendice C.

Parmi ces polydialkylsiloxanes, on peut citer à titre non limitatif les produits commerciaux suivants :
- les huiles SILBIONE® des séries 47 et 70 047 ou les huiles MIRASIL® commercialisées par RHODIA telles que, par exemple l'huile 70 047 V 500 000 ;
- les huiles de la série MIRASIL® commercialisées par la société RHODIA ;
- les huiles de la série 200 de la société DOW CORNING telles que la DC200 ayant viscosité 60 000 mm²/s ;
- les huiles VISCASIL® de GENERAL ELECTRIC et certaines huiles des séries SF (SF 96, SF 18) de GENERAL ELECTRIC.

La ou les huiles représentent généralement de 0 à 60 % en poids, et de préférence de 10 à 60 %, encore plus préférentiellement de 15 à 55 %, mieux de 20 à 50 % en poids par rapport au poids total de la composition selon l'invention.

La ou les huiles représentent généralement de 10 à 60 %, encore plus préférentiellement de 15 à 55 %, mieux de 20 à 50 % en poids par rapport au poids total de la composition selon l'invention.

Selon un mode de réalisation particulier, la composition conforme à l'invention comprend au moins 15 % en poids du poids total de la composition d'une ou plusieurs huiles différentes des alcools gras, de préférence au moins 20 % en poids.

Selon un mode de réalisation particulier, la composition conforme à l'invention ne comprend ni colorant ni persel.

Par milieu cosmétiquement acceptable, on entend, au sens de la présente invention, un milieu compatible avec les fibres kératiniques, et en particulier les fibres kératiniques humaines telles que les cheveux.

Le milieu cosmétiquement acceptable de la composition conforme à la présente invention comprend généralement de l'eau et / ou un ou plusieurs solvants organiques hydrosolubles. A titre de solvants organiques hydrosolubles, on peut par exemple citer les alcanols inférieurs en C₁-C₄, tels que l'éthanol et l'isopropanol ; les alcools aromatiques comme l'alcool benzylique ou le phénoxyéthanol ; les polyols ou éthers de polyols tels que les éthers monométhylique, monoéthylique et monobutylique d'éthylèneglycol, le propylèneglycol ou ses éthers tels que le monométhyléther de propylèneglycol, le butylèneglycol, le dipropylèneglycol ainsi que les alkyléthers de diéthylèneglycol comme le monoéthyléther ou le monobutyléther du diéthylèneglycol, ou encore le glycérol ; ainsi que leurs mélanges.

Les solvants sont, de préférence, présents dans des proportions comprises entre 0,1 et 35 % en poids environ par rapport au poids total de la composition oxydante, et encore plus préférentiellement entre 1 et 40 % en poids environ.

La composition conforme à l'invention peut également comprendre des composés additionnels utilisés classiquement en cosmétique. Ces composés peuvent notamment être choisis parmi les polymères épaississants ou stabilisants, les polymères conditionneurs non siliconés, les silicones, les chélatants, ainsi que les parfums.

Bien entendu, l'homme de l'art veillera à choisir ce ou ces éventuels composés complémentaires de manière telle que les propriétés avantageuses attachées intrinsèquement à la composition conforme à l'invention ne soient pas, ou substantiellement pas, altérées par la ou les adjonctions envisagées.

La composition selon l'invention peut se présenter sous des formes diverses, telles que sous forme de crème, de gel, de lait, de lotion, de mousse, ou sous toute notamment des fibres kératiniques humaines telles que les cheveux. De préférence, elle se présente sous la forme d'une crème ou d'un lait.

Le pH de la composition oxydante selon l'invention varie généralement de 1,5 à 4,5, et de préférence de 2 à 3,5. Il peut être ajusté par ajout d'agents acidifiants tels que l'acide chlorhydrique, l'acide acétique, l'acide lactique, l'acide borique, l'acide citrique et l'acide phosphorique ou d'agents acidifiants en présence d'agents alcalins.

Un autre objet de l'invention est un procédé de traitement des fibres kératiniques comprenant l'application sur les fibres kératiniques d'une composition oxydante telle que définie précédemment.

La composition oxydante conforme à l'invention peut par exemple être utilisée dans un procédé de teinture des fibres kératiniques, et en particulier des fibres kératiniques humaines telles que les cheveux.

Le procédé de teinture des fibres kératiniques conforme à l'invention met en oeuvre une composition colorante comprenant dans un support approprié pour la teinture des fibres kératiniques un ou plusieurs colorants directs et / ou un ou plusieurs colorants d'oxydation et une composition oxydante telle que définie ci-dessus.

Selon ce procédé, on applique sur les fibres kératiniques la composition colorante, la couleur étant révélée à pH acide, neutre ou alcalin à l'aide d'une composition oxydante selon l'invention qui est appliquée simultanément ou séquentiellement, avec ou sans rinçage intermédiaire.

Selon une forme de mise en oeuvre particulièrement préférée du procédé de teinture selon l'invention, on mélange, au moment de l'emploi, la composition colorante avec une composition oxydante selon l'invention. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

Le ou les colorants directs peuvent être choisis parmi les colorants directs classiquement utilisés en coloration directe. A titre d'exemples, ces colorants directs sont choisis parmi les colorants nitrés de la série benzénique, les colorants directs azoïques, les colorants directs méthiniques, les colorants directs quinoniques, les colorants directs aziniques, les colorants directs triarylméthaniques, les colorants directs indoaminiques, les colorants directs naturels. Ces colorants directs peuvent être de nature non ionique, anionique ou cationique.

Parmi les colorants directs benzéniques, on peut citer le 1,4-diamino-2-nitrobenzène, le 1-amino-2-nitro-4-(β-hydroxyéthylamino)-benzène, le 1-amino-2-nitro-4-bis(β-hydroxyéthyl)-aminobenzène, le 1,4-bis(β-hydroxyéthylamino)-2-nitro-benzène, le 1-β-hydroxyéthylamino-2-nitro-4-bis-(β-hydroxyéthylamino)-benzène, le 1-β-hydroxyéthylamino-2-nitro-4-amino-benzène, le 1-β-hydroxyéthylamino-2-nitro-4-(éthyl)(β-hydroxyéthyl)-aminobenzène, le 1-amino-3-méthyl-4-β-hydroxyéthylamino-6-nitro-benzène, le 1-amino-2-nitro-4-β-hydroxyéthylamino-5-chloro-benzène, le 1,2-diamino-4-nitro-benzène, le 1-amino-2-β-hydroxyéthylamino-5-nitro-benzène, le 1,2-bis-(β-hydroxyéthylamino)-4-nitro-benzène, le 1-amino-2-[tris-(hydroxyméthyl)-méthylamino]-5-nitro-benzène, le 1-hydroxy-2-amino-5-nitro-benzène, le 1-hydroxy-2-amino-4-nitro-benzène, le 1-hydroxy-3-nitro-4-amino-benzène, le 1-hydroxy-2-amino-4,6-dinitro-benzène, le 1-β-hydroxyéthyloxy-2-β-hydroxyéthylamino-5-nitro-benzène, le 1-méthoxy-2-p-hydroxyéthylamino-5-nitro-benzène, le 1-β-hydroxyéthyloxy-3-méthylamino-4-nitrobenzène, le 1-β,γ-dihydroxypropyloxy-3-méthylamino-4-nitrobenzène, le 1-β-hydroxyéthylamino-4-β,γ-dihydroxypropyloxy-2-nitro-benzène, le 1-β,γ-dihydroxypropylamino-4-trifluorométhyl-2-nitro-benzène, le 1-β-hydroxyéthylamino-4-trifluorométhyl-2-nitro-benzène, le 1-β-hydroxyéthylamino-3-méthyl-2-nitro-benzène, le 1-β-aminoéthylamino-5-méthoxy-2-nitro-benzène, le 1-hydroxy-2-chloro-6-éthylamino-4-nitro-benzène, le 1-hydroxy-2-chloro-6-amino-4-nitro-benzène, le 1-hydroxy-6-[bis-(β-hydroxyéthyl)-amino]-3-nitro-benzène, le 1-β-hydroxyéthylamino-2-nitro-benzène, le 1-hydroxy-4-β-hydroxyéthylamino-3-nitro-benzène.

Parmi les colorants directs azoïques, on peut citer les colorants azoïques cationiques décrits dans les demandes de brevets WO 95/15144, WO-95/01772, EP 0 714 954, FR 2 822 696, FR 2 825 702, FR 2 825 625, FR 2 822 698, FR 2 822 693, FR 2 822 694, FR 2 829 926, FR 2 807 650, WO 02/078660, WO 02/100834, WO 02/100369 et FR 2 844 269 dont le contenu fait partie intégrante de l'invention.

Parmi ces composés, on peut tout particulièrement citer le chlorure de 1,3-diméthyl-2-[[4-(diméthylamino)phényl]azo]-1H-Imidazolium, le chlorure de 1,3-diméthyl-2-[(4-aminophényl)azo]-1H-Imidazolium, le méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3^{e} édition : Disperse Red 17, Acid Yellow 9, Acid Black 1, Basic Red 22, Basic Red 76, Basic Yellow 57, Basic Brown 16, Acid Yellow 36, Acid Orange 7, Acid Red 33, Acid Red 35, Basic Brown 17, Acid Yellow 23, Acid Orange 24, Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques, on peut citer les colorants suivants : Disperse Red 15, Solvent Violet 13, Acid Violet 43, Disperse Violet 1, Disperse Violet 4, Disperse Blue 1, Disperse Violet 8, Disperse Blue 3, Disperse Red 11, Acid Blue 62, Disperse Blue 7, Basic Blue 22, Disperse Violet 15, Basic Blue 99, ainsi que les composés suivants : la 1-N-méthylmorpholiniumpropylamino-4-hydroxyanthraquinone, la 1-aminopropylamino-4-méthylaminoanthraquinone, la 1-aminopropylaminoanthraquinone, la 5-β-hydroxyéthyl-1,4-diaminoanthraquinone, la 2-aminoéthylaminoanthraquinone, la 1,4-bis-(β,γ-dihydroxypropylamino)-anthraquinone.

Parmi les colorants aziniques, on peut citer les composés suivants : Basic Blue 17, Basic Red 2.

Parmi les colorants triarylméthaniques, on peut citer les composés suivants : Basic Green 1, Acid blue 9, Basic Violet 3, Basic Violet 14, Basic Blue 7, Acid Violet 49, Basic Blue 26, Acid Blue 7.

Parmi les colorants indoaminiques, on peut citer les composés suivants : la 2-β-hydroxyéthlyamino-5-[bis-(β-4'-hydroxyéthyl)amino]anilino-1,4-benzoquinone, la 2-β-hydroxyéthylamino-5-(2'-méthoxy-4'-amino)anilino-1,4-benzoquinone, la 3-N(2'-Chloro-4'-hydroxy)phényl-acétylamino-6-méthoxy-1,4-benzoquinone imine, la 3-N(3'-Chloro-4'-méthylamino)phényl-uréido-6-méthyl-1,4-benzoquinone imine, la 3-[4'-N-(Ethyl,carbamylméthyl)-amino]-phényl-uréido-6-méthyl-1,4-benzoquinone imine.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine, les orcéines. On peut également utiliser les extraits ou décoctions contenant ces colorants naturels et notamment les cataplasmes ou extraits à base de henné.

Le ou les colorants directs sont généralement présents dans la composition colorante en quantité comprise entre 0,001 et 20 % en poids environ du poids total de la composition, et encore plus préférentiellement entre 0,005 et 10 % en poids environ.

Le ou les colorants d'oxydation peuvent être choisis parmi les bases d'oxydation et les coupleurs conventionnellement utilisés dans le domaine de la coloration.

A titre d'exemples de bases d'oxydation, on peut citer les para-phénylènediamines, les bases doubles, les para-aminophénols, les ortho-aminophénols, les bases hétérocycliques et leurs sels d'addition.

Parmi les para-phénylènediamines, on peut citer à titre d'exemple, la para-phénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylène-diamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition avec un acide.

Parmi les para-phénylènediamines citées ci-dessus, la para-phénylènediamine, la para-toluènediamine, la 2-isopropyl para-phénylènediamine, la 2-β-hydroxyéthyl para-phénylènediamine, la 2-β-hydroxyéthyloxy para-phénylène-diamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bases doubles, on peut citer, à titre d'exemples, les bis-phénylalkylènediamines et les bis-para-aminophénols.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition avec un acide ou avec une base.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2750048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

A titre de dérivés pyrazoliques, on peut aussi citer les diamino-N,N-dihydropyrazopyrazolones et notamment celles décrites dans la demande FR 2 886 136 telles que les composés suivants et leurs sels d'addition.

Parmi ces composés, les préférés sont les suivants :
2,3-Diamino-6,7-dihydro-1H,5H-pyrazolo[1,2-a]pyrazol-1-one,
2-Amino-3-éthylamino-6,7-dihydro-1H,5H-pyrazolo[1,2-a]pyrazol-1-one,
2-Amino-3-isopropylamino-6,7-dihydro-1H,5H-pyrazolo[1,2-a]pyrazol-1-one,
2-Amino-3-(pyrrolidin-1-yl)-6,7-dihydro-1 H,5H-pyrazolo[1,2-a]pyrazol-1-one,
4,5-diamino-1,2-diméthyl-1,2-dihydro-pyrazol-3-one,
4,5-diamino-1,2-diéthyl-1,2-dihydro-pyrazol-3-one,
4,5-diamino-1,2-di-(2-hydroxyéthyl)-1,2-dihydro-pyrazol-3-one,
2-amino-3-(2-hydroxyéthyl)amino-6,7-dihydro-1H,5H-pyrazolo[1,2-a]pyrazol-1-one,
2-amino-3-diméthylamino-6,7-dihydro-1H,5H-pyrazolo[1,2-a]pyrazol-1-one,
2,3-diamino-5,6,7,8-tétrahydro-1H,6H-pyridazino[1,2-a]pyrazol-1-one,
4-Amino-1,2-diethyl-5-(pyrrolidin-1-yl)-1,2-dihydro-pyrazol-3-one,
4-Amino-5-(3-dimethylamino-pyrrolidin-1-yl)-1,2-diethyl-1,2-dihydro-pyrazol-3-one,
2,3-diamino-6-hydroxy-6,7-dihydro-1H,5H-pyrazolo[1,2-a]pyrazol-1-one.

A titre de bases hétérocycliques, on utilisera préférentiellement le 4,5-diamino 1-(β-hydroxyéthyl) pyrazole, la 2,3-diamino-6,7-dihydro-1H,5H-pyrazolo[1,2-a]pyrazol-1-one et leurs sels d'addition.

La ou les bases d'oxydation sont généralement présentes dans la composition colorante en quantité comprise entre 0,001 et 10 % en poids environ du poids total de la composition, de préférence entre 0,005 et 6 % en poids environ.

A titre d'exemples de coupleurs, on peut citer les méta-phénylènediamines, les méta-aminophénols, les méta-diphénols, les coupleurs naphtaléniques, les coupleurs hétérocycliques et leurs sels d'addition.

On peut notamment citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Le ou les coupleurs sont généralement présents dans la composition colorante en quantité comprise entre 0,001 et 10 % en poids environ du poids total de la composition, de préférence entre 0,005 et 6 % en poids environ.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telle que la soude, la potasse, l'ammoniaque, les amines ou les alcanolamines.

La composition oxydante selon l'invention peut également être utilisée dans un procédé de décoloration des fibres kératiniques, et en particulier des fibres kératiniques humaines telles que les cheveux.

Le procédé de décoloration selon l'invention comprend une étape d'application sur les fibres kératiniques d'une composition décolorante comprenant de préférence de l'eau oxygénée en milieu alcalin après mélange extemporané. Classiquement, une deuxième étape du procédé de décoloration selon l'invention est une étape de rinçage des fibres kératiniques.

La composition décolorante appliquée sur les fibres kératiniques peut être obtenue par mélange d'une composition oxydante selon l'invention avec une composition aqueuse ou anhydre contenant de préférence un ou plusieurs agents alcalins. La composition anhydre peut être pulvérulente ou sous forme de pate et dans les deux cas contient de préférence un ou plusieurs sels peroxygénés, et en particulier un ou plusieurs persulfates. La composition anhydre sous forme de pate contient de plus un ou plusieurs liquides inertes organiques.

Un autre objet de la présente invention est un procédé de déformation permanente des fibres kératiniques, et en particulier des fibres kératiniques humaines telles que les cheveux, utilisant une composition oxydante telle que définie ci-dessus.

Selon ce procédé, on applique sur les fibres kératiniques à traiter une composition réductrice, les fibres kératiniques étant mises sous tension mécanique avant, pendant ou après l'application de la composition réductrice, on rince éventuellement les fibres, on applique sur les fibres éventuellement rincées la composition oxydante de la présente invention puis on rince éventuellement à nouveau les fibres.

La première étape de ce procédé consiste à appliquer sur les cheveux une composition réductrice. Cette application se fait mèche par mèche ou globalement.

La composition réductrice comprend au moins un agent réducteur, qui peut être en particulier choisi parmi l'acide thioglycolique, la cystéine, la cystéamine, le thioglycolate de glycérol, l'acide thiolactique, ou les sels des acides thiolactique ou thioglycolique.

L'habituelle étape de mise sous tension des cheveux sous une forme correspondant à la forme finale désirée pour ces derniers (boucles par exemple) peut être mise en oeuvre par tout moyen, mécanique notamment, approprié et connu en soi pour maintenir sous tension des cheveux, tels que par exemple rouleaux, bigoudis, peignes et analogues.

Les cheveux peuvent également être mis en forme sans l'aide de moyens extérieurs, simplement avec les doigts.

Avant de procéder à l'étape suivante facultative de rinçage, il convient, de manière classique, de laisser reposer pendant quelques minutes, généralement entre 5 minutes et une heure, de préférence entre 10 et 30 minutes, la chevelure sur laquelle a été appliquée la composition réductrice, et ceci de façon à bien laisser le temps au réducteur d'agir correctement sur les cheveux. Cette phase d'attente est effectuée de préférence à une température allant de 35 °C à 45 °C, en protégeant de préférence également les cheveux par un bonnet.

Dans la deuxième étape facultative de rinçage, les cheveux imprégnés de la composition réductrice sont rincés soigneusement par une composition aqueuse.

Puis, dans une troisième étape, on applique sur les cheveux ainsi rincés la composition oxydante selon la présente invention, dans le but de fixer la nouvelle forme imposée aux cheveux.

Comme dans le cas de l'application de la composition réductrice, la chevelure sur laquelle a été appliquée la composition oxydante est ensuite, de manière classique, laissée dans une phase de repos ou d'attente qui dure quelques minutes, généralement entre 3 et 30 minutes, de préférence entre 5 et 15 minutes.

Si la tension des cheveux était maintenue par des moyens extérieurs, on peut retirer de la chevelure ces derniers (rouleaux, bigoudis et analogues) avant ou après l'étape de fixation.

Enfin, dans la dernière étape du procédé selon l'invention, étape facultative également, les cheveux imprégnés de la composition oxydante sont rincés soigneusement, généralement à l'eau.

La présente invention a également pour objet l'utilisation pour le traitement des fibres kératiniques, et en particulier des fibres kératiniques humaines telles que les cheveux, d'une composition oxydante telle que définie ci-dessus.

La présente invention a notamment pour objet l'utilisation pour la teinture des fibres kératiniques, et en particulier des fibres kératiniques humaines telles que les cheveux, d'une composition oxydante telle que définie ci-dessus.

La présente invention a également pour objet l'utilisation pour la décoloration des fibres kératiniques, et en particulier des fibres kératiniques humaines telles que les cheveux, d'une composition oxydante telle que définie ci-dessus.

La présente invention a aussi pour objet l'utilisation pour la déformation permanente des fibres kératiniques, et en particulier des fibres kératiniques humaines telles que les cheveux, d'une composition oxydante telle que définie ci-dessus.

Les exemples suivants illustrent l'invention sans présenter un caractère limitatif.

### EXEMPLES

Les compositions suivantes sont préparées :

| Composition | C |
|---|---|
| Acide diéthylène triamine pentacétique, sel pentasodique en solution aqueuse à 40 % | 0,15 g |
| Peroxyde d'hydrogène en solution à 50 % (eau oxygénée 200 volumes) | 12 g |
| Stannate de sodium | 0,04 g |
| Pyrophosphate tétra-sodique, 10 H₂O | 0,03 g |
| Polycondensat tétraméthyl hexaméthylènediamine / dichloro 1,3-propylène en solution aqueuse vendu sous la dénomination MEXOMERE PO par la société Chimex | 0,25 g |
| Chlorure de poly di-méthyl di-allyl ammonium dans l'eau à 40 % non stabilisé vendu sous la dénomination Merquat 100 par la société Nalco | 0,50 g |
| Eau désionisée | 75,73 g |
| Huile de vaseline | 25 g |
| Glycérine | 0,50 g |
| Alcool stéarylique 30/70 | 8 g |
| Ceteareth-33 | 3 g |
| Amide d'acides de colza oxyéthyléné (4 OE) protégé PEG-4 RAPESEEDAMIDE | 1,30 g |
| Vitamine E | 0,10 g |
| Acide phosphorique q.s.^{.} | pH 2 |

La composition C est stable ;, y compris après 2 mois à 45 °C.

Des résultats analogues sont obtenus en remplaçant les 0,25 g de MEXOMERE PO et les 0,5 g de MERQUAT 100 par 0,75 g de MERQUAT 100 ou de MEXOMERE PO.

## Revendications

1. Composition pour le traitement des fibres kératiniques comprenant, dans un milieu cosmétiquement acceptable :
- un ou plusieurs polymères cationiques ;
- un ou plusieurs amides gras ;
- un ou plusieurs agents anti-oxygène choisis parmi la vitamine Ë, ses éthers ou ses esters ; et
- un ou plusieurs agents oxydants ;
le pH de cette composition étant compris entre 1,5 et 4,5,
la composition comprenant de plus au moins 10 % en poids du poids total de la composition d'une ou plusieurs huiles.

2. Composition pour le traitement des fibres kératiniques comprenant, dans un milieu cosmétiquement acceptable :
- un ou plusieurs polymères cationiques ;
- un ou plusieurs amides gras ;
- un ou plusieurs agents anti-oxygène choisis parmi la vitamine E, ses éthers ou ses esters ; et
- un ou plusieurs agents oxydants ;
le pH de cette composition étant alcalin,
la composition comprenant de plus au moins 10 % en poids du poids total de la composition d'une ou plusieurs huiles.

3. Composition selon la revendication 1 ou 2 dans laquelle le ou les polymères cationiques sont choisis parmi les polymères du type polyamine, polyaminoamide et polyammonium quaternaire.

4. Composition selon l'une quelconque des revendications 1 à 3 dans laquelle le ou les polymères cationiques sont choisis parmi le chlorure d'hexadiméthrine et les homo ou copolymères de chlorure de diméthyldiallylammonium.

5. Composition selon l'une quelconque des revendications précédentes dans laquelle le ou les amides gras sont choisis parmi les composés de formule (I) suivante : 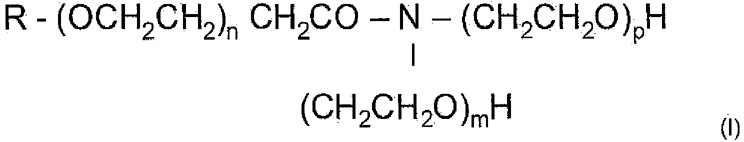 dans laquelle :
• R est un radical alkyle en C₈-C-₃₀, saturé ou insaturé, linéaire ou ramifié, éventuellement substitué par un ou plusieurs radicaux choisis dans le groupe constitué par un radical OR₅, un radical NR₆R₇, un radical carboxy, un radical sulfonique, un radical carboxamido CONR₆R₇, un radical sulfonamido SO₂NR₆R₇, un hétéroaryle, un aryle éventuellement substitué par un ou plusieurs groupes (C₁-C₄)alkyle, hydroxy, alcoxy en C₁-C₂, amino, (di)alkyl(C₁-C₂)amino ;
• R₅, R₆ et R₇, identiques ou différents, représentent :
- un atome d'hydrogène ;
- un radical alkyle linéaire ou ramifié en C₁-C₄ éventuellement substitué par un ou plusieurs radicaux choisis parmi un radical hydroxy, alcoxy en C₁-C₂, carboxamido CONR₈R₉, sulfonyle SO₂R₈, aryle éventuellement substitué par un radical (C₁-C₄)alkyle, hydroxy, alcoxy en C₁-C₂, amino, (di)alkyl(C₁-C₂)amino ; aryle éventuellement substitué par un radical (C₁-C₄)alkyle, hydroxy, alcoxy en C₁-C₂, amino, (di)atkyl(C₁-C₂)amino ;
• R₆ et R₇, identiques ou différents, peuvent représenter également un radical carboxamido CONR₈R₉ ; un radical sulfonyle SO₂R₈ ;
• R₈ et R₉, identiques ou différents, représentent un atome d'hydrogène ; un radical alkyle en C₁-C₄ linéaire ou ramifié éventuellement substitué par un ou plusieurs radicaux hydroxy, alcoxy en C₁-C₂ ;
• n, m et p, identiques ou différents, sont des nombres entiers compris entre 0 et 10, avec m + p > ou = 1.

6. Composition selon la revendication 5 dans laquelle R est choisi parmi un radical alkyle en C₁₀-C₁₈, n est un nombre entier allant de 0 à 5, m est un nombre entier allant de 0 à 1 et p est un nombre entier allant de 1 à 5.

7. Composition selon l'une quelconque des revendications 5 et 6 dans laquelle n est égal à 0.

8. Composition selon l'une quelconque des revendications précédentes dans laquelle l'amide gras est un amide d'acides de colza oxyéthyléné.

9. Composition selon l'une quelconque des revendications précédentes dans laquelle le ou les agents oxydants sont choisis parmi le peroxyde d'hydrogène, les persels, le peroxyde d'urée, les bromates alcalins, les polythionates.

10. Composition selon la revendication 9 dans laquelle l'agent oxydant est le peroxyde d'hydrogène.

11. Composition selon l'une des revendications précédentes, dans laquelle la ou les huiles sont choisies parmi les huiles non siliconées minérales, végétales, animales ou synthétiques.

12. Procédé de traitement des fibres kératiniques comprenant l'application sur les fibres kératiniques d'une composition oxydante telle que définie à l'une quelconque des revendications 1 à 11.

13. Utilisation pour le traitement des fibres kératiniques d'une composition oxydante telle que définie à l'une quelconque des revendications 1 à 11.

## Patentansprüche

1. Zusammensetzung zur Behandlung von Keratinfasern, die in einem kosmetisch unbedenklichen Medium Folgendes umfasst:
- ein oder mehrere kationische Polymere;
- ein oder mehrere Fettamide;
- ein oder mehrere Anti-Sauerstoff-Mittel, das/die aus Vitamin E, dessen Ethern und dessen Estern ausgewählt ist bzw. sind; und
- ein oder mehrere Oxidationsmittel;
wobei der pH-Wert dieser Zusammensetzung zwischen 1,5 und 4,5 liegt,
wobei die Zusammensetzung außerdem mindestens 10 Gew.-%, bezogen auf das Gesamtgewicht der Zusammensetzung, eines oder mehrerer Öle umfasst.

2. Zusammensetzung zur Behandlung von Keratinfasern, die in einem kosmetisch unbedenklichen Medium Folgendes umfasst:
- ein oder mehrere kationische Polymere;
- ein oder mehrere Fettamide;
- ein oder mehrere Anti-Sauerstoff-Mittel, das/die aus Vitamin E, dessen Ethern und dessen Estern ausgewählt ist bzw. sind; und
- ein oder mehrere Oxidationsmittel;
wobei der pH-Wert dieser Zusammensetzung alkalisch ist,
wobei die Zusammensetzung außerdem mindestens 10 Gew.-%, bezogen auf das Gesamtgewicht der Zusammensetzung, eines oder mehrerer Öle umfasst.

3. Zusammensetzung nach Anspruch 1oder 2, wobei das kationische Polymer bzw. die kationischen Polymere aus Polymeren vom Polyamin-, Polyaminoamid- und quaternären Polyammonium-Typ ausgewählt ist bzw. sind.

4. Zusammensetzung nach einem der Ansprüche 1 bis 3, wobei das kationische Polymer bzw. die kationischen Polymere aus Hexadimethrinchlorid und Homo- oder Copolymeren von Dimethyldiallylammoniumchlorid ausgewählt ist bzw. sind.

5. Zusammensetzung nach einem der vorhergehenden Ansprüche, wobei das Fettamid bzw. die Fettamide aus den Verbindungen der folgenden Formel (I) ausgewählt ist bzw. sind: 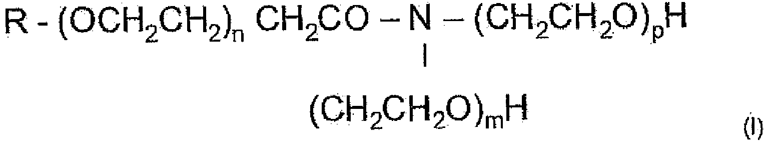 in der:
• R für einen gesättigten oder ungesättigten, linearen oder verzweigten C₈-C₃₀-Alkylrest, der gegebenenfalls durch einen oder mehrere Reste, die aus der Gruppe bestehend aus einem Rest OR₅, einem Rest NR₆R₇, einem Carboxyrest, einem Sulfonsäurerest, einem Carboxamidorest CONR₆R₇, einem Sulfonamidorest SO₂NR₆R₇, einem Heteroaryl, einem Aryl, das gegebenenfalls durch eine oder mehrere (C₁-C₄)Alkyl-, Hydroxy-, C₁-C₂-Alkoxy-, Amino-, (Di) alkyl (C₁-C₂) aminogruppen substituiert ist, ausgewählt sind, substituiert ist, steht;
• R₅, R₆ und R₇, die gleich oder verschieden sind, für:
- ein Wasserstoffatom;
- einen linearen oder verzweigten C₁-C₄-Alkylrest, der gegebenenfalls durch einen oder mehrere Reste, die aus einem Hydroxyrest, einem C₁-C₂-Alkoxyrest, einem Carboxamidorest CONR₈R₉, einem Sulfonylrest SO₂R₈, einem Arylrest, der gegebenenfalls durch einen (C₁-C₄)Alkyl-, Hydroxy-, C₁-C₂-Alkoxy-, Amino- oder (Di) alkyl (C₁-C₂) aminorest substituiert ist, ausgewählt sind, substituiert ist; einen Arylrest, der gegebenenfalls durch einen (C₁-C₄)Alkyl-, Hydroxy-, C₁-C₂-Alkoxy-, Amino-, (Di) alkyl (C₁-C₂)aminorest substituiert ist,
stehen;
• R₆ und R₇, die gleich oder verschieden sind, auch für einen Carboxamidorest CONR₈R₉, einen Sulfonylrest SO₂R₈ stehen können;
• R₈ und R₉, die gleich oder verschieden sind, für ein Wasserstoffatom; einen linearen oder verzweigten C₁-C₄-Alkylrest, der gegebenenfalls durch einen oder mehrere Hydroxy-, C₁-C₂-Alkoxyreste substituiert ist, stehen;
• n, m und p, die gleich oder verschieden sind, für ganze Zahlen zwischen 0 und 10 stehen, wobei m + p > oder = 1.

6. Zusammensetzung nach Anspruch 5, wobei R aus einem C₁₀-C₁₈-Alkylrest ausgewählt ist, n für eine ganze Zahl im Bereich von 0 bis 5 steht, m für eine ganze Zahl im Bereich von 0 bis 1 steht und p für eine ganze Zahl im Bereich von 1 bis 5 steht.

7. Zusammensetzung nach einem der Ansprüche 5 und 6, wobei n gleich 0 ist.

8. Zusammensetzung nach einem der vorhergehenden Ansprüche, wobei es sich bei dem Fettamid um ein oxyethyleniertes Amid von Rapssäuren, handelt.

9. Zusammensetzung nach einem der vorhergehenden Ansprüche, wobei das Oxidationsmittel bzw. die Oxidationsmittel aus Wasserstoffperoxid, Persalzen, Harnstoffperoxid, Alkalimetallbromaten, Polythionaten ausgewählt ist bzw. sind.

10. Zusammensetzung nach Anspruch 9, wobei es sich bei dem Oxidationsmittel um Wasserstoffperoxid handelt.

11. Zusammensetzung nach einem der vorhergehenden Ansprüche, wobei das Öl bzw. die Öle aus mineralischen, pflanzlichen, tierischen oder synthetischen Nichtsilikonölen ausgewählt ist bzw. sind.

12. Verfahren zur Behandlung von Keratinfasern, bei dem man auf die Keratinfasern eine oxidierende Zusammensetzung gemäß einem der Ansprüche 1 bis 11 aufbringt.

13. Verwendung einer oxidierenden Zusammensetzung gemäß einem der Ansprüche 1bis 11 zur Behandlung von Keratinfasern.

## Claims

1. Composition for the treatment of keratin fibres comprising, in a cosmetically acceptable medium:
- one or more cationic polymers;
- one or more fatty amides;
- one or more anti-oxygen agents chosen from vitamin E, its ethers or its esters; and
- one or more oxidizing agents;
the pH of this composition being between 1.5 and 4.5, the composition further comprising at least 10 wt.% of one or more oils, relative to the total weight of the competition.

2. Composition for the treatment of keratin fibres comprising, in a cosmetically acceptable medium:
- one or more cationic polymers;
- one or more fatty amides;
- one or more anti-oxygen agents chosen from vitamin E, its ethers or its esters; and
- one or more oxidizing agents;
the pH of this composition being alkaline,
the composition further comprising at least 10 wt.% of one or more oils, relative to the total weight of the competition.

3. Composition according to Claim 1 or 2, wherein the cationic polymer or polymers are selected from polymers of the polyamine, polyaminoamide and quaternary polyammonium type.

4. Composition according to either of Claims 1 to 3, wherein the cationic polymer or polymers are selected from hexadimethrine chloride and the homo- or copolymers of dimethyldiallylammonium chloride.

5. Composition according to any one of the preceding claims, wherein the fatty amide or amides are selected from the compounds of the following formula (I) : 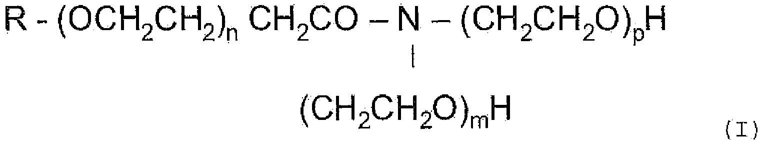 in which:
• R is a saturated or unsaturated, linear or branched C₈-C₃₀ alkyl radical, optionally substituted with one or more radicals selected from the group comprising a radical OR₅, a radical NR₆R₇, a carboxyl radical, a sulphonic radical, a carboxamido radical CONR₆R₇, a sulphonamido radical SO₂NR₆R₇, a heteroaryl, an aryl optionally substituted with one or more (C₁-C₄) alkyl, hydroxy, C₁-C₂ alkoxy, amino, (di) alkyl (C₁-C₂) amino groups;
• R₅, R₆ and R₇, which may be identical or different, represent:
- a hydrogen atom;
- a linear or branched C₁-C₄ alkyl radical optionally substituted with one or more radicals selected from a hydroxy, C₁-C₂ alkoxy, carboxamido CONR₈R₉, sulphonyl SO₂R₈, aryl radical optionally substituted with a (C₁-C₄)alkyl, hydroxy, C₁-C₂ alkoxy, amino, (di) alkyl (C₁-C₂)amino radical; aryl radical optionally substituted with a (C₁-C₄)alkyl, hydroxy, C₁-C₂ alkoxy, amino, (di)alkyl (C₁-C₂)amino radical;
• R₆ and R₇, which may be identical or different, can also represent a carboxamido radical CONR₈R₉; a sulphonyl radical SO₂R_{8;}
• R₈ and R₉, which may be identical or different, represent a hydrogen atom; a linear or branched C₁-C₄ alkyl radical optionally substituted with one or more hydroxyl, C₁-C₂ alkoxy radicals;
• n, m and p, which may be identical or different, are integers between 0 and 10, with m + p ≥ 1.

6. Composition according to Claim 5, wherein R is selected from a C₁₀-C₁₈ alkyl radical, n is an integer in the range from 0 to 5, m is an integer in the range from 0 to 1 and p is an integer in the range from 1 to 5.

7. Composition according to either of Claims 5 and 6, wherein n is equal to 0.

8. Composition according to any one of the preceding claims, wherein the fatty amide is an ethoxylated amide of colza acids.

9. Composition according to any one of the preceding claims, wherein the oxidizing agent or agents are selected from hydrogen peroxide, persalts, urea peroxide, alkaline bromates, polythionates.

10. Composition according to Claim 9, wherein the oxidizing agent is hydrogen peroxide.

11. Composition according to any one of the preceding claims, the composition further comprising at least 10 wt.% of one or more oils, relative to the total weight of the composition.
wherein the oil or oils are selected from the non-siliconized mineral, vegetable, animal or synthetic oils.

12. Method of treatment of keratin fibres, comprising the application of an oxidizing composition as defined in any one of Claims 1 to 11 on the keratin fibres.

13. Use of an oxidizing composition as defined in any one of Claims 1 to 11 for the treatment of keratin fibres.
